(19) **Europäisches Patentamt**
**European Patent Office**
**Office européen des brevets**

(11) **EP 1 583 970 B1**

(12) **EUROPÄISCHE PATENTSCHRIFT**

(45) Veröffentlichungstag und Bekanntmachung des Hinweises auf die Patenterteilung:
**15.08.2012 Patentblatt 2012/33**

(51) Int Cl.:
*G01N 33/68* *(2006.01)*    *G01N 33/573* *(2006.01)*

(21) Anmeldenummer: **03795945.9**

(22) Anmeldetag: **19.12.2003**

(86) Internationale Anmeldenummer:
**PCT/EP2003/014678**

(87) Internationale Veröffentlichungsnummer:
**WO 2004/057339 (08.07.2004 Gazette 2004/28)**

(54) **ELISA-VERFAHREN ZUM NACHWEIS VON GUANYLAT-BINDUNGSPROTEIN-1 (GBP-1)**

ELISA METHOD FOR THE DETECTION OF GUANYLATE BINDING PROTEIN 1 (GBP-1)

PROCEDE ELISA POUR LA DETECTION DE LA PROTEINE 1 DE LIAISON AU GUANYLATE (GBP-1)

(84) Benannte Vertragsstaaten:
**AT BE BG CH CY CZ DE DK EE ES FI FR GB GR HU IE IT LI LU MC NL PT RO SE SI SK TR**

(30) Priorität: **20.12.2002 DE 10260265**

(43) Veröffentlichungstag der Anmeldung:
**12.10.2005 Patentblatt 2005/41**

(73) Patentinhaber: **Helmholtz Zentrum München Deutsches Forschungszentrum für Gesundheit und Umwelt (GmbH) 85764 Neuherberg (DE)**

(72) Erfinder:
• **STÜRZL, Michael 91056 Erlangen (DE)**
• **LUBESEDER-MARTELLATO, Clara 80804 München (DE)**
• **GUENZI, Eric 85221 Dachau (DE)**
• **KREMMER, Elisabeth 85354 Freising (DE)**

(74) Vertreter: **Vossius & Partner Siebertstrasse 4 81675 München (DE)**

(56) Entgegenhaltungen:
**US-A1- 2002 115 138**

• **LUBESEDER-MARTELLATO CLARA ET AL: "Guanylate-binding protein-1 expression is selectively induced by inflammatory cytokines and is an activation marker of endothelial cells during inflammatory diseases" AMERICAN JOURNAL OF PATHOLOGY, Bd. 161, Nr. 5, November 2002 (2002-11), Seiten 1749-1759, XP009029105 ISSN: 0002-9440**
• **GUENZI E ET AL: "The helical domain of GBP-1 mediates the inhibition of endothelial cell proliferation by inflammatory cytokines" EMBO JOURNAL, OXFORD UNIVERSITY PRESS, SURREY, GB, Bd. 20, Nr. 20, 15. Oktober 2001 (2001-10-15), Seiten 5568-5577, XP002966002 ISSN: 0261-4189**
• **YIH SHYUN E ET AL: "Interferon induction of fibroblast proteins with Guanylate binding activity" JOURNAL OF BIOLOGICAL CHEMISTRY. (MICROFILMS), AMERICAN SOCIETY OF BIOLOGICAL CHEMISTS, BALTIMORE, MD, US, Bd. 258, Nr. 12, 23. Juni 1983 (1983-06-23), Seiten 7746-7750, XP002128621**
• **STREHLOW INGA ET AL: "The interferon-inducible GBP1 gene: Structure and mapping to human chromosome 1" GENE (AMSTERDAM), Bd. 144, Nr. 2, 1994, Seiten 295-299, XP001180829 ISSN: 0378-1119**

## Beschreibung

Die vorliegende Erfindung betrifft Verfahren zur Identifizierung und/oder Quantifizierung von GBP-1 oder von Fragmenten dieses Proteins im Kulturüberstand einer Gewebeprobe, einer Probe von Körperflüssigkeit oder einer Probe eines Zellkulturüberstandes.

Das Endothel ist ein Schlüsselorgan bei zahlreichen physiologischen und pathophysiologischen Prozessen wie Zell-gesteuerter Immunantwort, Menstruation, Wundheilung, Entzündung, Allergie, Herz-Kreislauferkrankung und Tumorwachstum. Die Pathofunktion des Endothels ist untrennbar mit der Aktivierung von Endothelzellen verbunden.

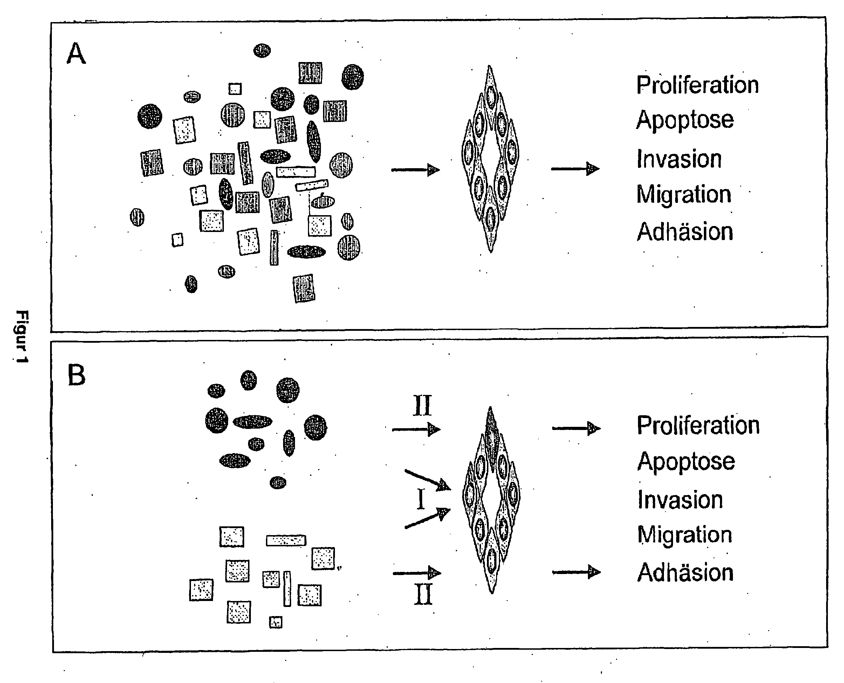

Die Aktivierung des Endothels ist ein komplexer Vorgang, der durch eine Vielzahl verschiedener löslicher Faktoren gesteuert wird, die im Blut zirkulieren oder von benachbarten Zellen freigesetzt werden (Fig. 1A). In Folge dieses Prozesses werden die Physiologie und Morphologie der Endothelzellen an die jeweiligen Erfordernisse im Gewebe angepasst. Im Vordergrund stehen hierbei die Steuerung der Zellproliferation, Apoptose, Invasion, Migration und Leukozyten-Adhäsionsfähigkeit von Endothelzellen, wodurch die Neu- und Rückbitdung von Gefäßen und die Extravasation von Leukozyten reguliert werden (Fig. 1A).

Die Vielzahl der beteiligten Faktoren legt nahe, dass mehrere Faktoren den selben Phänotyp steuern und zu wirkungsgleichen Gruppen zusammengefaßt werden können (Fig. 1B). Zum Beispiel aktivieren die angiogenen Wachstumsfaktoren *basic fibroblast growth factor* (bFGF) und *vascular endothelial cell growth factor* (VEGF) die Endothelzellproliferation, wohingegen die inflammatorischen Zytokine Interleukin (IL)-1$\alpha$, IL-1$\beta$, *tumor necrosis factor* (TNF)-$\alpha$ und Interferon (IFN)-$\gamma$ die Proliferation hemmen und die Leukozyten-Adhäsionsfähigkeit von Endothelzellen erhöhen.

Derzeit gibt es keine geeigneten Methoden, mit denen bestimmt werden kann, wo und wann die verschiedenen Faktoren in entzündlichen Geweben auf die Endothelzellen wirken. Daher ist über die räumliche und zeitliche Verteilung der verschiedenen Aktivierungszustände von Endothelzellen im Rahmen entzündlicher Erkrankungen nur sehr wenig bekannt.

Die Entwicklung von Entzündungsreaktionen und sich daraus ergebender entzündlicher Erkrankungen ist eine sehr komplexe Abfolge (Kaskade) von unterschiedlichen und synergistischen Wirkungen von inflammatorischen Faktoren wie Zytokinen, die eine Analyse eines definierten Stadiums einer Entzündungsreaktionen und/oder eine verlässliche Voraussage über deren weitere Entwicklung schwer möglich machen. Aufgrund dieser Komplexheit der ablaufenden Reaktionen spricht man in diesem Zusammenhang auch von sogenannten Zytokinnetzwerk.

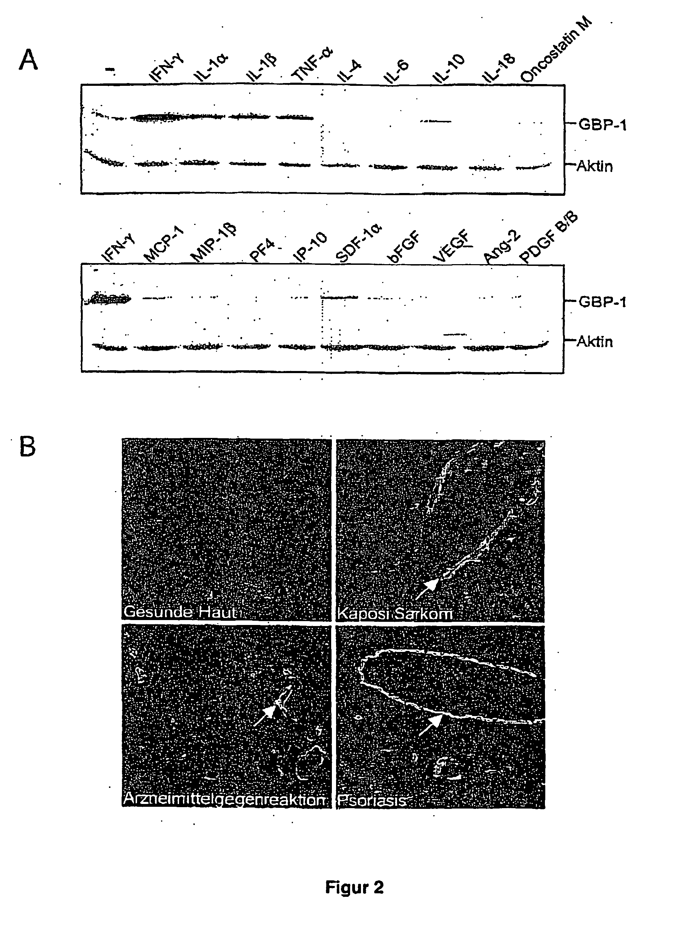

Erste Arbeiten, einen molekularen Marker zu identifizieren, der eine Aktivierung von Endothelzellen durch die oben genannten inflammatorischen Zytokine im Gewebe anzeigt, führten zu vergleichenden Untersuchungen zur Genexpression in kultivierten Endothelzellen in Gegenwart unterschiedlicher Aktivierungsbedingungen. Mit diesem Ansatz konnte ein Gen isoliert werden, dessen Expression in Endothelzellen selektiv durch inflammatorische Zytokine induziert wird (Fig. 2A) (Guenzi et al., 2001; Lubeseder-Martellato et al. 2002). Dieses Gen kodiert für das Guanylat-Bindungsprotein-1 (GBP-1), das zur Proteinfamilie der großen GTPasen gehört.

Um zu bestimmen, ob GBP-1 wie in kultivierten Zellen auch bei Gefäßendothelzellen in humanen Geweben eine Aktivierung durch inflammatorische Zytokine anzeigt, wurden immunhistochemische Untersuchungen zum Nachweis von GBP-1 mittels spezifischer monoklonaler Antikörper durchgeführt. Dazu wurden histologische Schnitte gesunder Haut und von Hauterkrankungen mit einer entzündlichen Komponente, wie zum Beispiel Psoriasis, Arzneimittelgegenreaktion und Kaposi-Sarkom untersucht (Fig. 2B). Allen genannten Hauterkrankungen ist gemeinsam, dass in den Läsionen fokal konzentriert zahlreiche Entzündungszellen vorliegen, die dieselben inflammatorischen Zytokine freisetzen, die auch zu einer erhöhten GBP-1-Expression führen. In den Gefäßen der gesunden Haut konnte GBP-1 in keinem Fall nachgewiesen werden. Im Gegensatz dazu waren in allen untersuchten entzündlichen Erkrankungen einzelne Gefäße deutlich positiv für GBP-1 (Fig. 2B, Pfeile). Diese Ergebnisse zeigten, dass GBP-1 tatsächlich auch in humanen Geweben eine inflammatorische Aktivierung von Endothelzellen anzeigt und als molekularer Marker für den Nachweis dieser Aktivierung in Geweben eingesetzt werden kann (Lubeseder-Martellato et al. 2002, Guenzi et al. 2001). Diese Nutzung als molekularer Marker war jedoch auf feste Gewebeproben beschränkt, da die beschriebenen Ergebnisse zeigten, das das Protein GBP-1 ein in der Zelle wirkendes Protein ist, das im Zytoplasma der Zelle lokalisiert ist. Entsprechend umfassten entsprechende Nachweise die z.B. die Gewinnung von festen Gewebeproben von Patienten. Eine Entnahme von festen Gewebeproben aus entzündlichem Gewebe geht jedoch mit nachteiligen Effekten für die Patienten einher und ist schwierig.

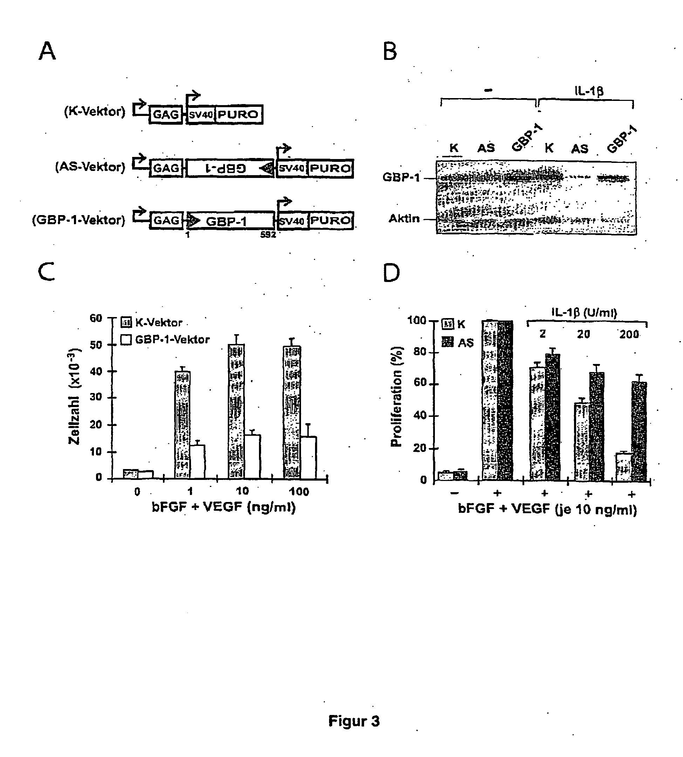

Die Induktion der GBP-1-Expression durch inflammatorische Zytokine geht in Endothelzellen mit einer Hemmung der Zellproliferation einher. Daher wurde untersucht, ob GBP-1 die durch inflammatorische Zytokine induzierte Proliferationshemmung vermittelt. Dazu wurden Endothelzellen mit retroviralen Vektoren transduziert, welche die konstitutive Expression von GBP-1 (GBP-1-Vektor) oder einer *antisense*-GBP-1-RNA (AS-Vektor) bewirken (Abb. 3A). Western blot-Analysen belegten, dass Endothelzellen die mit dem GBP-1-Vektor transduziert wurden, GBP-1 sehr stark exprimierten (Fig. 3B). In Zellen, die mit dem AS-Vektor transduziert wurden, war die Induktion der GBP-1-Expression durch IL-1$\beta$ effizient blockiert (Fig. 3B). Nachfolgende Proliferationsexperimente mit den verschiedenen transduzierten Zellkulturen zeigten, dass GBP-1 tatsächlich die durch angiogene Wachstumsfaktoren induzierte Zellproliferation hemmt (Fig. 3C, weiße Balken) und darüber hinaus notwendig ist, dass inflammatorische Zytokine die Proliferation von Endothelzellen hemmen können (Fig. 3D). Letzteres äußert sich darin, dass in *antisense*-GBP-1-RNA exprimierenden Zellkulturen die Hemmwirkung inflammatorischer Zytokine auf die Zellproliferation deutlich herabgesetzt ist (Fig. 3D, schwarze Balken) (siehe Guenzi et al. 2001).

Darüber hinaus hemmt GBP-1 die Expression von Matrix-Metalloproteinase-1 und damit einhergehend die Invasion von Endothelzellen (siehe Guenzi et al., 2003).

Weiterführende Untersuchungen zur Struktur-/Funktionsbeziehung von GBP-1 zeigten, dass interessanterweise die Adhäsionsfähigkeit von Endothelzellen für Leukozyten, die ebenfalls durch inflammatorische Zytokine induziert wird, durch GBP-1 nicht beeinflusst wird (Guenzi et al. 2001). GBP-1 ist somit ein neuer, molekularer Marker für eine entzündliche Gefäßaktivierung, der selektiv die antiproliferative Wirkung inflammatorischer Zytokine auf Endothelzellen steuert.

Bislang konnte gezeigt werden, dass GBP-1 selektiv durch inflammatorische Zytokine induziert wird und dass dieser Prozeß mit einer anti-angiogenen Wirkung auf die betreffenden Zellen korreliert (Lubeseder-Martellato et al., 2002 und Guenzi et al., 2001). Während man sich die Induktion von GBP-1 über dessen antiproliferative Wirkung im Prinzip für medizinische Zwecke im Rahmen einer anti-angiogenen Therapie zu Nutze machen könnte, ist der Einsatz inflammatorischer Zytokine für derartige Zwecke aufgrund der pleiotropen und damit auch nachteiligen Effekte dieser Zytokine indiskutabel.

Obwohl ein Bedarf an geeigneten molekularen Markern für inflammatorische Erkrankungen besteht, sind eine Vielzahl von Zytokinen und Faktoren, die an der Entstehung und Entwicklung von inflammatorischen Erkrankungen beteiligt sind, im Stand der Technik aufgrund Ihrer Instabilität als für diesen Zweck ungeeignet beschrieben. Darüber hinaus ist eine Quantifizierung einzelner inflammatorischer Zytokine oder Faktoren nicht ausreichend für einen eindeutigen Befund und erfordert deshalb die Bestimmung einer Vielzahl unterschiedlicher Zytokine und Faktoren, die nur in einem sogenannten "Zytokinnetzwerk" ihre jeweilige Wirkung zeigen.

Aufgabe der vorliegenden Erfindung war somit Verfahren bereitzustellen, welches eine einfache und gezielte Analyse der Expression von GBP-1 ermöglicht. Diese Verfahren sollte Aussagen über das Stadiums und Fortschreiten einer Entzündungsreaktion in einem Individuum oder in einem in vitro Modell ermöglicht ohne dass eine aufwendige Analyse und Quantifizierung von vielen verschiedenen inflammatorischen Faktoren des sogenannten Zytokinnetzwerks notwendig ist.

Diese Aufgabe wird durch die Bereitstellung der in den Ansprüchen gekennzeichneten Ausführungsformen gelöst.

Folglich betrifft die vorliegende Erfindung ein in den Ansprüchen definiertes in vitro Verfahren zur Identifizierung und/oder Quantifizierung von GBP-1 oder von Fragmenten dieses Proteins im Kulturüberstand einer Gewebeprobe, einer Probe von Körperflüssigkeit oder einer Probe eines Zellkulturüberstandes, wobei das Verfahren die folgenden Schritte umfasst:

(a) In Kontakt bringen der Probe mit einem ersten Rezeptor, der GBP-1 spezifisch bindet; und

(b) Nachweis einer spezifischen Bindung des Rezeptors mit GBP-1 oder einem Fragmente dieses Proteins.

Der Begriff Fragment von GBP-1 beschreibt Fragmente des Proteins, welche die biologische Aktivität von GBP-1 besitzen wie im Stand der Technik wie auch in dieser Anmeldung beschrieben. Erfindungsgemäß der Bagriff Fragmente des Proteins, die durch Spaltung, z.B. enzymatische Spaltung, entstehen und indikativ für inflammatorische Erkrankungen sind.

Der Begriff Körperflüssigkeiten umfasst im Zusammenhang mit der Erfindung alle Arten von Körperflüssigkeiten, ggf. verdünnt oder aufkonzentriert. Beispiele sind Blut/Serum, Plasma, Fruchtwasser, Hirn-Rückenmarkflüssigkeit, Liquor, Zerebrospinalflüssigkeit, Sputum, Rachen und Schlund-Sekrete und andere Schleimhautsekrete, Synovialflüssigkeit, Ascites, Tränenflüssigkeit, Lymphflüssigkeit und Urin.

Der Begriff der "spezifische Bindung" beschreibt erfindungsgemäß eine spezifische Interaktion oder Wechselwirkung zwischen einem Rezeptor und einem Liganden. Ein solche Ligand ist GBP-1 oder Fragmente dieses Proteins. Die spezifische Interaktion oder Wechselwirkung kann mit einem "Schlüssel-Schloß-Prinzip" charakterisiert werden. Der Rezeptor und der Ligand besitzen Strukturen oder Motive, die spezifisch zueinander passen, wie z.B. eine antigene Determinante (Epitop), die mit der Antigen-Bindungstelle eines Antikörpers wechselwirkt. Entsprechend steht spezifische Interaktion im Gegensatz zu einer universelleren, unspezifischen Wechselwirkung.

Es wurde gezeigt, dass GBP-1 ein Markerprotein für Entzündungsreaktionen ist, dass überraschenderweise u.a. von endothelialen Zellen und Monozyten sezemiert wird. Durch diesen überraschenden Befund wird es möglich GBP-1 im Kulturüberstand von Gewebeproben, Proben von Körperflüssigkeit oder Proben von Zellkulturüberständen zu analysieren und eine Aussage über das Stadium einer entzündlichen Erkrankung zu treffen. Sezerniertes GBP-1 kann mit dem erfindungsgemäßen Verfahren einfach und schnell nachgewiesen werden und dient somit als krankheitsassoziierter diagnostischer Parameter.

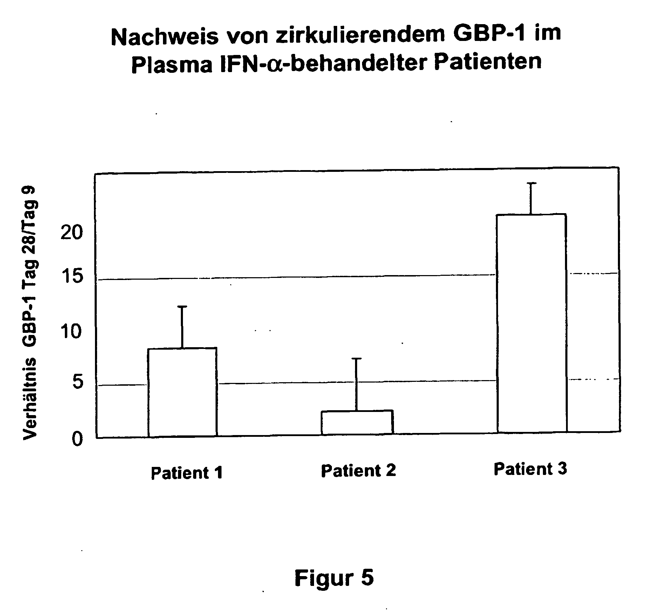

Der Nachweis einer entzündlichen Aktivierung von Endothelzellen und Monozyten in der Körperflüssigkeit von Patienten mit dem erfindungsgemäßen Verfahren ist auf Grundlage des überraschenden Befundes von besonderer Bedeutung bei Entzündungserkrankungen, bakteriellen und viralen Infektionserkrankungen (AIDS, Menigitis), Allergien, Transplantationsreaktionen, Herz-Kreislauf- und Tumorerkrankungen usw. Darüber hinaus sind diese von Bedeutung für die Bestimmung der Ansprechreaktion bei Patienten unter der Behandlung mit inflammatorischen Zytokinen (z.B. Interferon-$\alpha$); siehe Figur 5.

Der Nachweis, bzw. die quantifizierte Menge von GBP-1 in einer Probe einer Körperflüssigkeit eines Patienten ermöglicht Rückschlüsse auf den Aktivierungsgrad von Endothelzellen und Monozyten und damit eine Aussage über das Krankheitsbild des Patienten.

Verfahren zur Gewinnung von den genannten Proben sind dem Fachmann bekannt. Optional umfasst das erfindungsgemäße Verfahren darüber hinaus einen oder mehrere Waschschritte vor oder nach jedem Verfahrensschritt. Diese Waschschritte dienen der Minimierung von unspezifischen Reaktionen (falsch positiver oder falsch negativer Nachweis) und können die Sensitivität des Verfahrens verbessern. Geeignete Waschpuffer und deren Zusammensetzung sind dem Fachmann im Prinzip bekannt; siehe z.B. Harlow und Lane. Bevorzugt sind physiologische Pufferlösungen.

Eine bevorzugte Ausführungsform des erfindungsgemäßen Verfahrens umfasst darüber hinaus den Schritt (a') oder (a") vor dem in Kontakt bringen mit dem ersten Rezeptor:

(a') Markieren der in der Probe enthaltenen Proteine; oder

(a") Markieren des ersten Rezeptors.

Die in der Probe enthaltenen Proteine und/oder der erste Rezeptor können beispielsweise chemisch markiert werden, z.B. durch die Kopplung von markierten chemischen Gruppen oder Markern an die freien Aminogruppen von in den Proteinen enthaltenen Cysteine. Beispiele für solche markierte chemische Gruppen sind Gruppen, die spezielle, nachweisbare Radioisotope enthalten. Als Marker können z.B. auch Fluoreszenzfarbstoffe dienen. Ein weiteres Beispiel für entsprechende Marker stellen Nukleinsäuren dar. Die Anwesenheit von auf diese Weise markierten Proteinen oder Rezeptoren in einer Probe kann dann mit geeigneten Primern in einer Polymerasekettenreaktion (PCR) nachgewiesen werden.

Des weiteren ist es möglich Proteine physiologisch zu markieren, d.h. durch den metabolischen Einbau von markierten Molekülen. Zu diesem Zweck werden Zellen beispielsweise mit radioaktiv markierten Metaboliten inkubiert. Proteine, die während dieser Inkubationszeit aus der Biosynthese dieser Zellen hervorgehen und in welche die markierten Metaboliten eingebaut wurden, sind makiert. Dieses Verfahren ist z.B. geeignet, um von antikörperproduzierenden Zellen sezernierte Antikörper zu markieren.

In einer weiteren bevorzugten Ausführungsform des erfindungsgemäßen Verfahrens wird der Rezeptor vor dem in Kontakt bringen mit GBP-1 oder von Fragmenten dieses Proteins auf einer Oberfläche immobilisiert. Entsprechend einer alternativen Ausführungsform des erfindungsgemäßen Verfahrens wird der Rezeptor nach dem in Kontakt bringen mit GBP-1 oder von Fragmenten dieses Proteins auf einer Oberfläche immobilisiert.

Rezeptoren können auf vielfältige Weise immobilisiert werden. Das entsprechende Verfahren hängt von verschiedenen Faktoren ab, wie z.B. von der Art des Rezeptors oder dem Material der Oberfläche. Eine Immobilisierung kann kovalent oder durch Adsorption erfolgen. Entsprechend einer bevorzugten Ausführungsform des erfindungsgemäßen Verfahrens sind die Rezeptoren Proteine, besonders bevorzugt Antikörper. Ebenso bevorzugt ist auch die Verwendung von Peptiden oder organischen Molekülen als Rezeptoren.

Für die Immobilisierung von Rezeptoren, die Proteine sind, werden Verfahren beschrieben, bei welchen diese direkt auf einer Oberfläche mittels passiver Adsorption immobilisiert werden. Üblicherweise besteht eine entsprechende Oberfläche aus einem polymeren Kunststoff (z.B. Polystyrol, Polyvinyl, Latex) und z.B. in Form von Mikrotiterplatten oder Multi-well-Platten, Membranen oder sphärischen 'Beads' (quervernetzten Polymeren in Partikelform) für diesen Zweck verwendet (Lowman, Annu. Rev. Biophys. Biomol. Struct. 26 (1997), 401-24).

In einer weiter bevorzugten Ausführungsform des erfindungsgemäßen Verfahrens ist das Material der Oberfläche ausgewählt aus einer Gruppe bestehend aus Sepharose, Latex, Glass, Polystyrol, Polyvinyl, Nitrocellulose und Silicium.

Weiter bevorzugt ist die Oberfläche in dem erfindungsgemäßen Verfahrens eine Membran, ein Kügelchen, ein Chip oder eine Platte.

Beispiele für Kügelchen sind Sepharose-beads oder Latex-beads, an die optional Liganden gebunden sind, die eine Immobilisierung der Rezeptoren an die Oberfläche begünstigen. Solche Liganden sind beispielsweise Protein-A oder Protein-G, die eine Bindung von Antikörper an eine Oberfläche über den Fc-Teil der Antikörper begünstigen. Die Bindung des Rezeptors an Trägermaterial kann auch durch eine kovalente chemische Kopplungsreaktion (z.B. Hydrazid-Kopplung) erreicht werden. Beispiel 3 beschreibt ein entsprechendes Verfahren. Ein anderes Beispiel für die Immobilisierung der Rezeptoren an die Oberfläche unter Verwendung von Liganden ist die Verwendung von Biotin und Avidin, bzw.

Streptavidin.

Beispiele für Chips sind Siliziumplatten, auf die eine Vielzahl von verschiedenen oder gleichen Rezeptoren geordnet imobilisiert werden kann. Dies ermöglicht die Analyse einer Vielzahl von unterschiedlichen Parametern in einer Probe oder die Analyse einer Vielzahl von unterschiedlichen Proben auf einen oder mehrere Parameter hin, z.B. Identifizierung und/oder Quantifizierung von GBP-1 oder von Fragmenten dieses Proteins in unterschiedlichen Gewebeproben, Proben von Körperflüssigkeit oder Proben von Zellkulturüberstanden.

Beispiele für die genannten Platten sind Mikrotiterplatten oder Multi-well-Platten. Diese besitzen vorzugsweise 6, 12, 24, 48, 96, 128, 356, 1024 oder mehr Vertiefungen. In Beispiel 4 ist ein Verfahren beschrieben, in dem 96well-Platten verwendet werden.

Entsprechend einer weiter bevorzugten Ausführungsform des Verfahrens umfasst dieses darüber hinaus den Schritt (a''') vor dem Schritt des Nachweises einer spezifischen Bindung:

(a''') präzzipitieren der Kügelchen mit den daran gebundenen Komplexen aus erstem Rezeptor und GBP-1 oder eines Fragmentes dieses Proteins Kügelchen können aus einer Probe z.B. gravimetrisch präzipitiert werden. Dies kann beispielsweise durch Zentrifugation beschleunigt werden. Entsprechende Verfahren sind dem Fachmann u.a. aus Rehm, Der Experimentator: Proteinbiochemie/Proteomics, Spektrum Akademischer Verlag, 2002 bekannt. Des weiteren wird eine entsprechende Präzipitation in Beispiel 3 beschrieben.

In einer weiter bevorzugten Ausführungsform des erfindungsgemäßen Verfahrens umfasst der Nachweis der spezifischen Bindung in Schritt (b) eine gelelektrophoretische Auftrennung, optional darüber hinaus eine Western-Blot-Analyse (siehe Beispiel 3). Entsprechende Verfahren sind dem Fachmann u.a. aus Rehm, loc. cit. bekannt.

In einer weiter bevorzugten Ausführungsform des erfindungsgemäßen Verfahrens wird zum Nachweis einer spezifischen Bindung von GBP-1 oder eines Fragmentes dieses Proteins an den ersten Rezeptor in Schritt (a) die Probe mit einem zweiten Rezeptor für GBP-1 in Kontakt gebracht, der an ein Epitop von GBP-1 oder eines Fragmentes dieses Proteins bindet, das nach Bindung des ersten Rezeptors an GBP-1 oder eines Fragmentes dieses Proteins zugänglich ist.

Diese bevorzugt Ausführungsform betrifft beispielsweise Verfahren, die sich das mechanistische Prinzip des Sandwich-ELISA's zu Nutze machen. Dieses Prinzip ist dem Fachmann allgemein bekannt und wird u.a. in Stryer, Biochemie, Spektrum Akademischer Verlag, 1996 beschrieben. Ein entsprechendes Verfahren ist darüber hinaus im beigefügten Beispiel 4 beschrieben.

Weiter ist der zweite Rezeptor für GBP-1 oder Fragmente dieses Proteins in einer bevorzugten Ausführungsform des erfindungsgemäßen Verfahrens markiert. Verfahren, die eine Markierung eines Rezeptors ermöglichen, wurden oben beschrieben und können auch hier eingesetzt werden.

Darüber hinaus ist bevorzugt, dass die Markierung des zweiten Rezeptors für GBP-1 oder eines Fragmentes dieses Proteins ein signalgebendes System umfasst. Ebenso bevorzugt ist eine spezifische Erkennung der Markierung durch einen weiteren, dritten Rezeptor, der ein signalgebendes System umfasst.

Ein Beispiele für ein solches signalgebendes System ist die oben beschriebene Isotopenmarkierung, wobei das Signal die Abgabe von radioaktiver Strahlung ist. Ebenso resultiert eine Fluoreszenzmarkierung des entsprechenden Rezeptors in der Markierung mit einem signalgebenden System im Sinne der Erfindung, wobei das Signal die Emission eines Fluoreszenzsignals nach entsprechender Anregung des Farbstoffes ist.

Weiter bevorzugt umfasst das signalgebende System erfindungsgemäß ein Enzym, das ein Signal abgibt. Beispiele für solche Enzyme umfassen alkalische Phosphatasen, Peroxidasen, β-Galaktosidase, Glucoamylase, Urease und Chloramphenikol-Azetyltransterase. Entsprechende Beispiele und der Einsatz notwendiger Substrate für den Nachweis mit Hilfe von enzymatischen Reaktionen sind dem Fachmann bekannt, u.a. aus den Beipackzetteln kommerzielle erhältlichen Nachweiskits oder aus Rehm, loc. cit. Solche kommerzielle erhältlichen Nachweiskits enthalten oft Antikörper, die Antikörper bestimmter Spezies erkennen, z.B. anti-Maus, und an die signalgebende Enzyme gekoppelt sind. Somit stellen entsprechende Antikörper ein Beispiel für einen dritten Rezeptor dar, die eine bestimmte Markierung des zweiten Rezeptors, nämlich dessen Fc-Teil erkennen.

Erfindungsgemäß ist der erste und der zweite Rezeptor und der dritte Rezeptor ausgewählt aus der Gruppe bestehend aus Antikörpern oder unten definierten davon und Aptameren.

Der Begriff Peptide bezeichnet üblicherweise Aminosäureketten mit bis zu 30 Aminosäuren. Der Begriff Polypeptide bezeichnet Peptide, die üblicherweise mehr als Aminosäureketten 30 Aminosäuren umfassen und schließt Proteine ein. Unter dem Begriff "niedermolekulare Substanzen" oder kleine Moleküle werden Moleküle verstanden, die von geringerer molekularer Komplexität sind, als die oben definierten Makromoleküle. In der Literatur wird der Begriff "niedermolekulare Substanzen" nicht einheitlich verwendet. In WO 89/03041 und WO 89/03042 werden Moleküle mit Molekülmassen bis 7000 g/mol als kleine Moleküle beschrieben. Üblicherweise werden jedoch Molekülmassen zwischen 50 und 3000 g/mol, häufiger aber zwischen 75 und 2000 g/mol und meistens im Bereich zwischen 100 und 1000 g/mol angegeben. Beispiele sind dem Fachmann aus den Schriften WO86/02736, WO97/31269, US-A-5928868, US-A-5242902, US-A-5468651, US-A-5547853, US-A-5616562, US-A-5641690, US-A-4956303 und US-A-5928643 bekannt. Niedermolekulare Substanzen können organischer oder anorganischer Natur sein.

Der Begriff "Antikörper" umfasst erfindungsgemäß polyklonale Sera, wie auch monolonale Antikörper.

Monoklonale Antikörper und Verfahren zu deren Herstellung sind dem Fachmann bekannt. Diese basieren auf einer zuerst von Köhler und Milstein (1975) beschriebenen Methode. Diese ist u.a. in dem Laborhandbuch von Harlow und Lane (Antibodies, A laboratory manual; Cold Spring Harbor Laboratory; (1988); Chapter 6) detailliert beschrieben. Durch die Definition umfaßt sind ebenfalls bispezifische Antikörper, synthetische Antikörper und Fragmente dieser Antikörper. Diese sind die Fragmente Fab, Fv oder scFv und chemisch modifizierte Derivate dieser Antikörper oder Antikörperfragmente.

Aptamere sind dem Fachmann dem Fachmann im Prinzip aus dem Stand der Technik bekannt.

Vorzugsweise ist das erfindungsgemäße Verfahren ein ELISA, EIA, oder RIA.

Entsprechende Verfahren sind dem Fachmann im Prinzip bekannt aus Harlow und Lane, loc. cit. und Rehm, loc. cit.

Das erfindungsgemäße Verfahren wird vorzugsweise automatisiert ausgeführt. Dies ist u.a. möglich durch die Verwendung von Pipetierrobotem und für eine automatisierte Auswertung optimierter Arbeitsgänge.

Ferner betrifft die Erfindung die Verwendung von Körperflüssigkeit oder einer Probe eines Zellkulturüberstandes, wie vorstehend definiert, zum Nachweis von GBP-1 oder von Fragmenten dieses Proteins, wobei der positive Nachweis indikativ ist für das Vorhandensein einer inflammatorischen Erkrankung.

Die weiteren (bevorzugten) Ausführungsformen der erfindungsgemäßen Verwendung entsprechen den für das oben beschriebene Verfahren.

Die Figuren zeigen:

### Fig. 1: Komplexität und Redundanz entzündlicher Endothelzellaktivierung.

**(A)** Die Aktivierung des Endothels bei entzündlichen Prozessen wird durch eine Vielzahl verschiedener löslicher Faktoren aus dem Blut und von benachbarten Zellen gesteuert. Dabei stehen im Rahmen der Neu- und Rückbildung von Gefäßen, sowie der Extravasation von Leukozyten die Steuerung von Zellproliferation, Apoptose, Invasion, Migration und Adhäsionsfähigkeit für Leukozyten im Vordergrund.

**(B)** Die Vielzahl der beteiligten Faktoren legt nahe, dass mehrere Faktoren ein- und dieselbe Aktivierung beeinflussen und zu wirkungsgleichen Gruppen zusammengefasst werden können. Gegenwärtig kann nicht bestimmt werden, wann und wo die verschiedenen Faktoren auf die einzelnen Endothelzellen einwirken. Darüber hinaus sind die Beziehungen der meisten Aktivierungsarten zueinander weitgehend unbekannt. Es ist zu bestimmen, ob alle Aktivierungen gleichzeitig in einer Zelle auftreten können (I) oder aufgrund zellbiologischer Restriktionen zeitlich, beziehungsweise räumlich separiert sein müssen (II).

### Fig.2: Expression von GBP-1 in kultivierten Endothelzellen und in entzündlichen Erkrankungen der Haut.

**(A)** Western Blot-Analyse der GBP-1 Expression in Endothelzellen, die mit den aufgeführten Faktoren für 24 h stimuliert worden waren. Folgende Konzentrationen wurden eingesetzt: IFN-$\gamma$ (100 U/ml), IL-1$\alpha$ (5 ng/ml), IL-1$\beta$ (200 U/ml), TNF-$\alpha$ (300 U/ml), IL-4 (10 U/ml), IL-6 (50 U/ml), IL-10 (50 ng/ml), IL-18 (100 ng/ml), Oncostatin M (10 ng/ml), MCP-1 (50 ng/ml), PF4 (25 ng/ml), SDF-1$\alpha$ (200 ng/ml), bFGF (10 ng/ml), VEGF (10 ng/ml), Ang-2 (800 ng/ml) und PDGF B/B (100 ng/ml). Der gleichzeitige Nachweis des Zytoskelettproteins Aktin zeigt, dass gleiche Proteinmengen aufgetragen wurden.

**(B)** Induktion der GBP-1-Expression in vaskulären Endothelzellen bei Hauterkrankungen mit einer inflammatorischen Komponente. Indirekte Immunfloreszenzfärbung von GBP-1 (grün) und dem Endothelzell-assoziierten Antigen CD31 (rot) in Gewebeschnitten von gesunder Haut, Kaposi-Sarkom, entzündlicher Arzneimittelgegenreaktion der Haut und Psoriasis. Die Überlagerung der Bilder zeigt eine Ko-Expression (gelb) von GBP-1 und CD31 (weiße Pfeile). (Modifiziert nach (Lubeseder-Martellato, Guenzi et al. 2002))

### Fig.3: GBP-1 vermittelt die antiproliferative Wirkung inflammatorischer Zytokine in Endothelzellen.

**(A)** Schematische Darstellung des retroviralen Expressionsvektors pBabePuro (Kontrollvektor, K-Vektor) in den die cDNA von GBP-1 in beiden Orientierungen für die konstitutive Expression von GBP-1 (GBP-1-Vektor) und für die Expression einer GBP-1-*antisense*-RNA (AS-Vektor) eingesetzt wurde.

**(B)** GBP-1-Expression in K-, GBP-1-, und AS-Vektor transduzierten Endothelzellen, die entweder unbehandelt waren oder über einen Zeitraum von 24 h mit 20 U/ml IL-1$\beta$ stimuliert wurden. Der Nachweis der GBP-1-Expression erfolgte mittels WesternBlot-Analyse mit einem polyklonalen anti-GBP-1-Antikörper. Die gleichzeitige Färbung mit Aktin zeigt, dass gleiche Proteinmengen aufgetragen wurden.

**(C)** Proliferationsexperimente mit K-Vektor- und GBP-1-Vektor-transduzierten Endothelzellen in Anwesenheit steigender Konzentrationen angiogener Wachstumsfaktoren (bFGF und VEGF in Kombination).
**(D)** Proliferationsexperimente von K-Vektor- und AS-Vektor-transduzierten Endothelzellen in Anwesenheit angiogener Wachstumsfaktoren und aufsteigender Konzentrationen von IL-1β. (Modifiziert nach (Guenzi, Topolt et al. 2001))

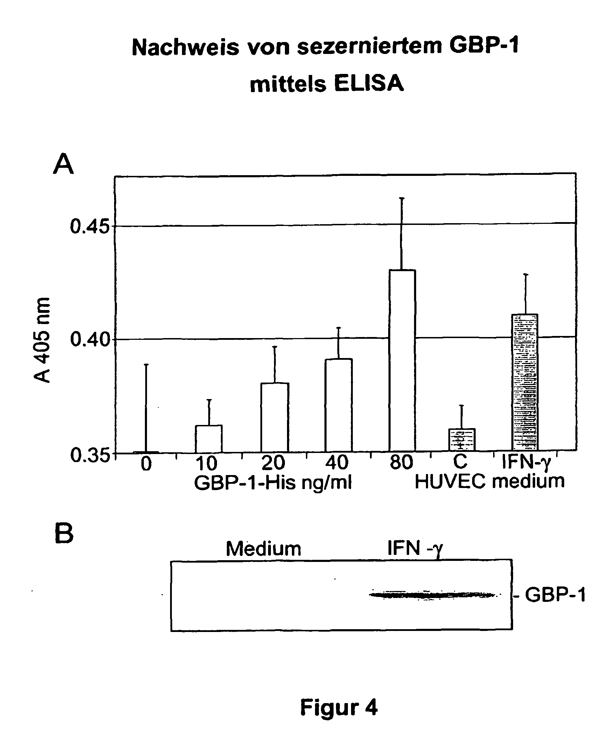

**Fig. 4: Nachweis von GBP-1 Protein in Kulturmedium von IFN-γ stimulierten HUVEC durch ELISA und Immunopräzipitation**
HUVEC wurden Stimuliert mit 100 U/ml IFN-γ (IFN-γ) oder unbehandelt gelassen (Medium). Nach 24 h Kultur wurde das Kulturmedium durch ELISA (A) oder Immunopräzipitation (B) analysiert.

**(A)** Eine Verdünnungsserie mit rekombiantem aufgereinigtem GBP-1 wurde als Standard verwendet (weiße Säulen). Die Menge von sekretiertem GBP-1 Protein wurde im ELISA bestimmt (graue Säulen). Die Absorption wurde bei 405 nm bestimmt.
**(B)** Westernblot-Analyse von immunopräzipitierten menschlichem GBP-1 Protein mit monoklonalem anti GBP-1 Antikörper (Klon 1B1) aus dem selben Kulturüberstand wie in (A).

**Fig. 5: Messung von zirkulierendem GBP-1 im Plasma von IFN-α behandelten Patienten**
Bei Melanom-Patienten, die für 9 bzw. 28 Tage mit IFN-α behandelt wurden, wurde die Konzentration von zirkulierendem GBP-1 im Plasma mittels ELISA bestimmt. Dargestellt ist der Anstieg der GBP-1 Konzetration von Tag 9 zum Tag 28 bei drei Pateinten. ELISA-Mikrotiterplatten wurden mit einem monoklonalen Ratten anti-GBP-1 Antikörper, Klon 1B1, für 16 h bei 4°C beschichtet. Anschließend wurden die Platten mit PBS-T (0,1 % Tween 20 in PBS) und für 30 min wurden bei Raumtemperatur (RT) freie Bindungsstellen mit PBS-T/BSA 2 % (PBS-TB) abgesättigt. Nach dem absaugen des PBS-TB erfolgte Inkubation (2 h) mit je 100 μl verschiedener Plasma Proben (1:2) bei RT. Als Standard wurde gereinigtes GBP-1-His Protein, verdünnt in Zellkulturmedium (EMB-0,5% FCS) benutzt. Als Negativkontrolle diente BSA. Die Proben wurden 4x mit PBS-T gewaschen, je 100 μl polyklonaler Kaninchen anti-GBP-1 Antikörper (1:500) zugegeben und für 2 h bei RT inkubiert. Nach viermaligen Waschen mit PBS-T erfolgte eine Inkubation (1 h,RT) mit je 100 μl AP-konjugiertem anti-Kaninchen Antikörper (1:500 verdünnt in PBS-TB). Nach vier weiteren Waschschritten wurde der GBP-1 Protein/Antikörper-Komplex durch Inkubation mit 100 μl p-Nitrophenyl Phosphat visualisiert. Die Absorption wurde bei 405 nm im Mikroplatten-Reader gemessen. Die Konzentrationsbestimmung erfolgte anhand einer Standardkurve für die steigende Konzentrationen von gereinigtem GBP-1-His verwendet wurden. Sie Linearität der Messung ist für einen Bereich von 0,1 bis 100 ng/ml gegeben.

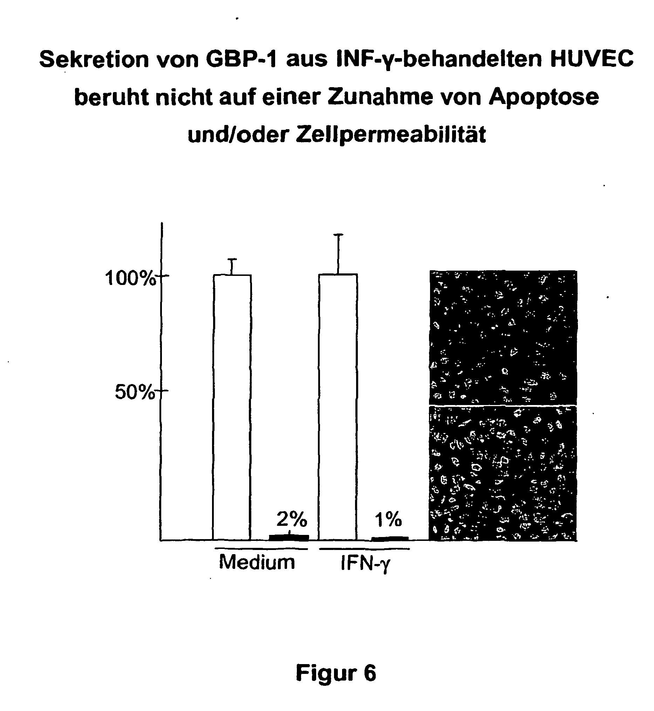

**Fig. 6: Die Sekretion von GBP-1 Protein aus IFN-γ behandelt HUVEC ist nicht zunehmende Zellmembranpermeabilität oder Apoptose zurückzuführen**
Färbung von HUVEC mit dem nicht membrangängigen Farbstoff „Dead-Red" (Molekular Probes).
HUVEC wurden über Nacht in 0,5 % FBS-haltigen EBM-Medium kultiviert und mit 100 U/ml Interferon-γ (IFN-γ, Roche) stimuliert. Kontrollzellen (Medium) wurden unbehandelt belassen. 24 h nach der Stimulierung wurden die Zellen mit *Dead-Red* Farbstoff angefärbt. Zellen, die eine veränderte Membranpermeabilität aufweisen (schwarze Balken), sind als Prozentsatz der Gesamtzahl (weiße Balken) angegeben.

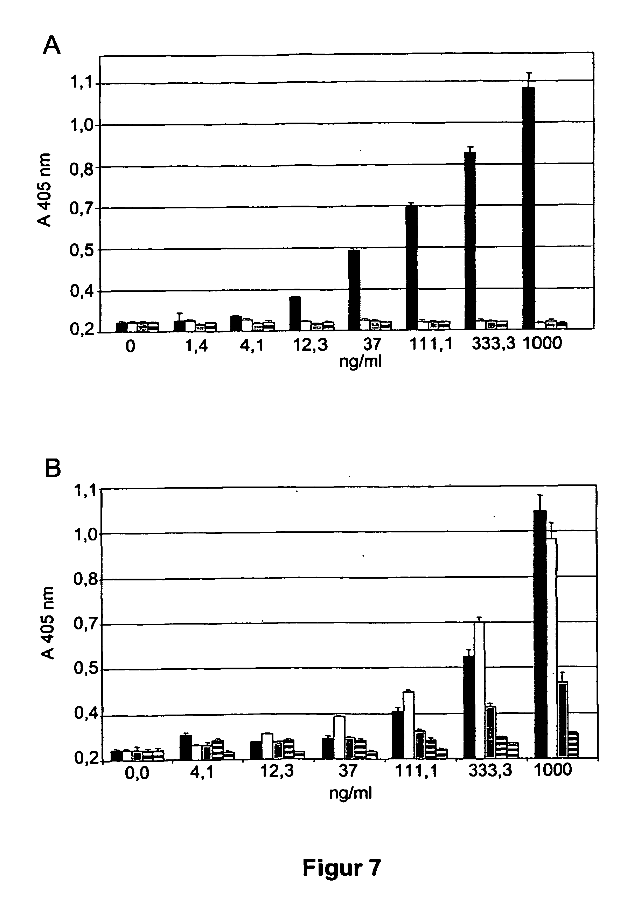

**Fig. 7. Der ELISA reagiert spezifisch mit GBP-1 und nicht mit den heterologen Proteinen BSA und eGFP**

**(A)** Die Spezifät des GBP-1-ELISA wurde in mehreren Kontrollexperimenten bestimmt. Zunächst wurde mit dem ELISA eine Verdünnungsreihe von gereinigtem GBP-1-His (ein GBP-1-Protein, das aus Bakterien gereinigt wurde und zum Zwecke der Aufreinigung mit 6 Histidinresten am Carboxyterminus versehen ist) in PBS in den angegebenen Konzentrationen erzeugt und im ELISA gemessen. Hierbei wurde ein konzentrationsabhängiger Anstieg der Absorption bei 405 nm ($A_{405}$) beobachtet (schwarze Balken). Wenn anstelle des anti-GBP-1-Kaninchenserums (wird als zweiter Antikörper zum Nachweis des gebundenen GBP-1 eingesetzt) ein Präimmunserum des Kaninchens verwendet wurde, wurden keine Signale erhalten (weiße Balken). Beim Zusatz aufsteigender Konzentrationen heterologer Proteine [BSA (graue Balken), His-eGFP (gestreifte Balken)] wurden mit dem GBP-1-spezifischen ELISA ebenfalls keine Signale beobachtet.
**(B)** Der GBP-1-ELISA zeigt eine geringe Reaktivität mit dem GBP-1-homologen GBP-2.

Mit dem GBP-1-ELISA wurden Lösungen untersucht, die aufsteigende Mengen an GBP-1-His (schwarze Balken), His-GBP-1 (weiße Balken, ein GBP-1-Protein, das aus Bakterien gereinigt wurde und zum Zwecke der Aufreinigung mit 6 Histidinresten am Aminoterminus versehen ist) und His-GBP-2 [graue Balken, einem zu GBP-1 homologen Protein (Homologie auf Aminosäureebene 76 %, auf Nukleinsäureebene 82 %) mit 6 Histidinresten am Aminoterminus] enthielten. Der Vergleich der Steigungen der Messwerte für GBP-1-His (schwarze Balken), His-GBP-1 (weiße Balken) und His-GBP-2 (graue Balken) zeigte, dass die Reaktivität dieses ELISA mit GBP-2 im Vergleich zur Reaktivität mit GBP-1 verringert ist und das die Histidinreste am Amino- oder Carboxyterminus die Reaktivität von rekombinantem GBP-1 nicht beeinflussen.

Zusätzlich wurden immunchemische Blockierungsversuche durchgeführt, um die Spezifität der Bindung von gereinigtem GBP-1-His and die mit Ratten-anti-GBP-1-Antikörpern beschichtete ELISA-Platte zu bestimmen. Hierzu wurde GBP-1-His in verschiedenen Verdünnungen mit (schwarz-weiße Balken) oder ohne (schwarze Balken) Ratten-anti-GBP-1-Antiköper inkubiert und nachfolgend auf die Platte gegeben. In den Ansätzen, in denen GBP-1-His nicht mit Antikörpern vorinkubiert wurde, wurde eine konzentrationsabhängige Erhöhung der Signalstärke beobachtet (schwarze Balken). Wohingegen, durch Vorinkubation mit anti-GBP-1-Antikörpern (schwarz-weiße Balken) die Bindung von GBP-1 an die Plattenoberfläche blockiert werden konnte. In einem vergleichbar durchgeführten Ansatz konnte auch die Bindung von His-GBP-2 durch Vorinkubation mit dem Ratten-anti-GBP-1-Antikörper blockiert werden (grau-weiße Balken).

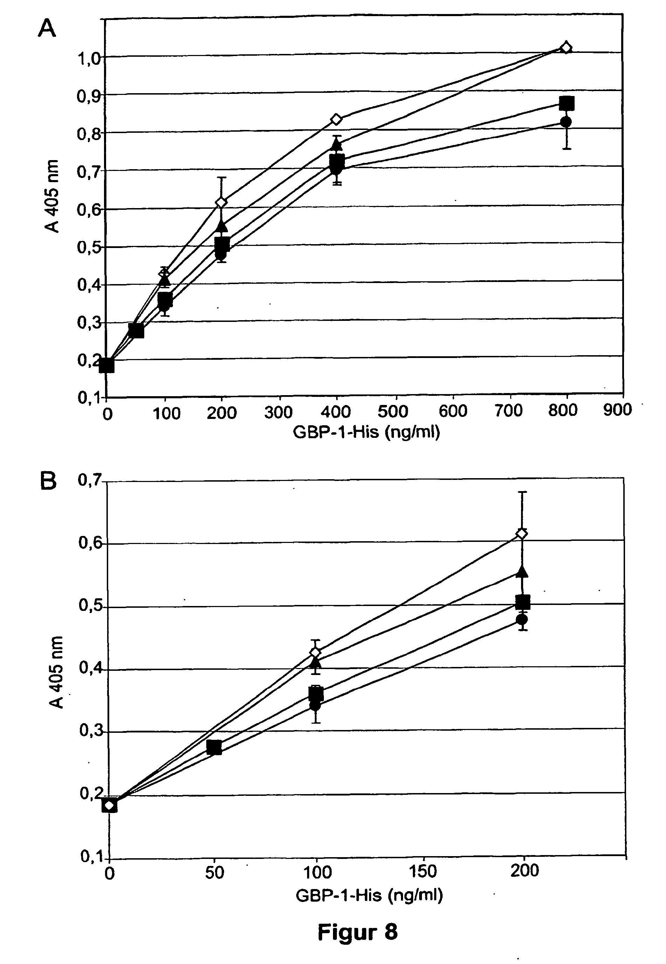

**Fig. 8: Bestimmung des linearen Nachweisbereichs des ELISA**

(A) Gepoolte Seren gesunder Personen wurden 1:2 (Quadrate), 1:4 (Kreise), 1:8 (Dreiecke) und 1:16 (Rauten) in PBS/2% BSA verdünnt. In die unterschiedlichen Verdünnungen wurden jeweils aufsteigende Konzentrationen von GBP-1-His zugegeben. Nachfolgend wurden alle Proben mit dem GBP-1-ELISA vermessen. Es zeigte sich, dass bei jeder der unterschiedlichen Serumverdünnungen die Nachweissignale (A405) mit der GBP-1-Konzentration zunahmen.

(B) Zwischen 0 und 200 ng/ml wurde bei den in (A) beschriebenen Ansatz eine lineare Zunahme des Signals beobachtet.

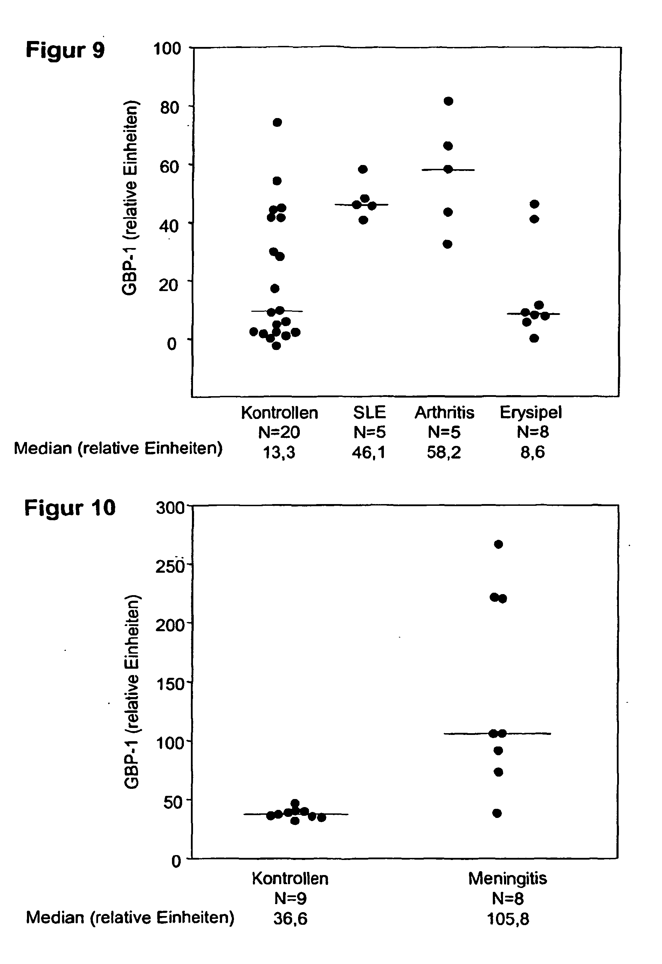

**Fig.9: Bei Patienten mit Entzündungserkrankungen sind erhöhte GBP-1 Konzentrationen im Serum nachweisbar**

Die GBP-1 Konzentration wurde mittels ELISA in gesunden Kontrollpersonen (n = 20) und in Seren von Patienten mit verschiedenen Entzündungserkrankungen (n = 10) gemessen: Systemischer Lupus Erythematosus (SLE) (n = 5) und Arthritis (n = 5), diese Erkrankungen sind generalisierte Entzündungen, und in Patienten mit Erysipel (n=8), eine örtlich begrenzte Entzündung der Haut.

Die GBP-1 Serumkonzentrationen wurden bestimmt, wobei die Serum-Proben 1:2 verdünnt wurden. Die Konzentration von GBP-1 in den Proben wurde mit Hilfe einer Standardkurve berechnet. Da das ELISA-Verfahren auch GBP-2 erkennt und möglicherweise auch GBP-2 im Serum vorhanden ist, wurden die ermittelte Konzentrationen in relativen Einheiten angegeben.

Die GBP-1 Serumkonzentrationen waren deutlich erhöht bei den Patienten mit SLE (Median: 46,1 relative Einheiten) und Arthritis (Median: 58,2 relative Einheiten) aber nicht in Patienten mit Erysipel (Median: 8,6 relative Einheiten) im Vergleich zu den Kontrollpersonen (Median: 13,3 relative Einheiten). Die Unterschiede der GBP-1 Konzentrationen bei Patienten mit SLE und Arthritis und der GBP-1 Konzentration in gesunden Kontrollpersonen sind statistisch signifikant (Wilcoxon-Test $p < 0,01$ für SLE und Arthritis).

**Fig.10: Bei Patienten mit bakterieller Meningitis sind erhöhte GBP-1-Konzentrationen im Liquor nachweisbar**

In einem Blindversuch wurde mittels ELISA die GBP-1 Konzentration in 17 Liquor-Proben nachgewiesen (Figur 10). Nach Entblindung des Versuches zeigte sich, dass bei Patienten mit bakterieller (Pneumokokkus, Staphylokokkus aureus, Pseudomonas aeruginosa) Meningitis (n = 8) im Vergleich zu gesunden Kontrollpersonen (n = 9) signifikant erhöhte (Wilcoxon-Test $p<0,03$) GBP-1 Konzentrationen im Liquoren nachweisbar waren (Kontrollpersonen = 36,6 relative Einheiten und bakterieller Meningitis = 105,8 relative Einheiten). Die GBP-1 Liquorkonzentrationen wurden bestimmt wie beschrieben. Die Liquor-Proben wurden 1:2 in PBS verdünnt. Die Konzentration von GBP-1 in der Probe wurde mit Hilfe einer Standardkurve berechnet. Da das ELISA-Verfahren auch GBP-2 erkennt und möglicherweise auch GBP-2 im Liquor vorhanden ist, wurden die ermittelte Konzentrationen in relativen Einheiten angegeben.

Die GBP-1 Konzentration im Liquor gesundern Kontrollpersonen zeigten keinen statisch signifikanten Unterschied (Wilcoxon-Test $p>0,05$) zu den GBP-1 Konzentrationen im Serum. Dies weist darauf hin, dass GBP-1 nicht im Liquor gesunder Parsonen angereichert ist und dass der Anstieg der GBP-1 Konzentrationen bei Patienten mit Meningitis krankheitsbedingt ist.

Die Beispiele erläutern die Erfindung.

**Beispiel 1: Herstellung eines Vektorkonstruktes**

Das 237 bp GBP-1 Promotorfragment (pro237-GBP-1) wurde mittels PCR-Amplifikation (PCR 2 Advantage Kit, Clontech) von dem Konstrukt pro3757-GBP-1 mit den Oligonukleotiden 5'-ATTTG<u>AAGCTT</u>CTGGTTGAG-3' [einfü-

gen einer HindIII-Schnittstelle (unterstrichen)] bzw. 5'-TGGCTTCTAGCACTTCTG-3' generiert. Das Konstrukt pro3757-GBP-1 enthält 3757 bp der 5'-regulatorischen Sequenz aufwärts des ATG-Kodons des *GSP-1* Gens (gi:4503938, NM_ 002053) im Vektor pT-Adv (Clontech). Das 237 bp Fragment wurde in Antisense-Orientierung mit dem Vektor pT-Adv ligiert, mit HindIII geschnitten und in den pGL3-Basic Vektor (Promega) subkloniert. Alle Konstrukte wurden mit dem Endofree Maxi Kit (Qiagen) aufgereinigt und zur Verifizierung sequenziert.

### Beispiel 2: Etablierung einer geeigneten Zellinie

HEK 293 T-Zellen (humane embryonische epitheliale Nierenzellinie mit humanen Adenovirus Typ 5 (Ad 5) DNA transformiert (ATCC CRL 1573), welche zusätzlich mit SV40 T Antigen transformiert sind) wurden mit $3x10^5$ Zellen/ Well (6-Multi-Well Platte, Corning) 24 h vor der Transfektion ausgesät. Pro Well der 6-Well Platte wurden gesamt 0,8 $\mu$g Plasmid verwendet, wobei das Testplasmid pro237-GBP-1 im Verhältnis 1:5 mit dem Selektionsplasmid pBABE-Puro (P. Monini, Laboratory of Virology, Instituto Superiore di Santiä, Rome, Italien) eingesetzt wurde. Das Testplasmid pro237-GBP-1 enthält das 237 bp Promotorfragment gekoppelt an das Indikatorgen firefly-Luziferase, das Selektionsplasmid pBABE-Puro das Resistenzgen Puromycin, worauf anschließend selektiert wird. Die Transfektion der Zellen wurde analog Vorschrift des Herstellers mit Effectene (Qiagen) durchgeführt und nach 24 h die Selektion mit 0,7 $\mu$g/ml Puromycin (Sigma) begonnen. Bei Tag 8-10 konnte das Wachstum von Einzelklonen beobachtet werden, die anschließend mit Klonierringen trypsiniert und in Einzelwells einer 96-well Platte (Falcon) überführt wurden. Bei entsprechender Konfluenz wurden die Klone weiter passagiert und auf ihre Reportergen-aktivität im Luziferaseassay untersucht. Die Klone wurden mit inflammatorischen Zytokinen IFN-$\gamma$, IL-1$\beta$ und TNF-$\alpha$, sowie Buffer für 5 h stimuliert und in 1x "passive lysis buffer" (Promega) abgeerntet. Die Lysate wurden auf firefly-Luziferase-Aktivität untersucht und entsprechend stabile Klone etabliert.

### Beispiel 3: Immunopräzipitation von GBP-1

Frisch hergestellte Zelllysate wurden vorgereinigt durch Inkubation mit 2 $\mu$l nicht mit GBP-1 reagierendem Präimmunserum von Kaninchen und 25 $\mu$l Protein A/G Agarosebeads für mindestens 3 h bei 4°C auf einer Schüttelplättform. Nach dem Pelletieren der Beads wurde der Überstand mit 25 $\mu$l Protein A/G Agarosebeads und 1 $\mu$l von polyklonalem Kaninchenserum gegen GBP-1 über Nacht bei 4°C auf einer Schüttelplattform inkubiert. Die Beads wurden vier bis fünf mal in PBS gewaschen. Anschließend wurden die Beads in 30 $\mu$l Laemmli-Probenpuffer (2X) resuspendiert und für 5 min gekocht. Die Proben wurden in einer SDS-PAGE (10%) aufgetrennt und in einem Westernblot oder in einer Autoradiographie analysiert. Für die Immunopräzipitation von GBP-1 oder MMP-1 aus Zellkulturüberständen wurden 10 ml Kulturmedium auf Eis gestellt, 5 min zentrifugiert bei 1000 rpm, filtriert durch einen Filter mit einer Porengröße von 45 $\mu$m und in einigen Fällen ein Proeinaseninhibitor-Cocktail (0,02 mg/ml Pankreasextrakt, 5 $\mu$g/ml Pronase, 0,5 $\mu$g/ml Thermolysin, 3 $\mu$g/ml Chymotrypsin und 0,33 jm/ml Papin) zugegeben. Die Vorreinigung wurde ausgeführt durch Inkubation mit 10 $\mu$l Kaninchenserum und 120 $\mu$l Protein A/G Agarosebeads für mehr als 3 h bei 4°C auf einer Schüttelplattform. Nach dem Pelletieren der Beads wurde der Überstand mit 120 $\mu$l Protein A/G Agarosebeads und 6 $\mu$l von polyklonalem Kaninchenserum gegen GBP-1 über Nacht bei 4°C auf einer Schüttelplattform inkubiert. Die Beads wurden vier bis fünf mal in PBS gewaschen. Anschließend wurden die Beads in 60 $\mu$l PBS + 60 $\mu$l Laemmli-Probenpuffer (2X) resuspendiert und für 5 min gekocht. Üblicherweise wurden 15 $\mu$l jeder Probe in einer SDS-PAGE (10%) aufgetrennt und in einem Westernblot analysiert.

Ein Beispiel für das Ergebnis eines Nachweises von GBP-1 durch Immunopräzipitation aus Kulturmedium ist in Figur 4B dargestellt.

### Beispiel 4: GBP-1 ELISA

Für den entwickelten GBP-1 ELISA wurden folgende Puffer verwendet:

PBS enthaltend 0, 1 % Tween 20 (PBS-T) und PBS enthaltend 0, 1 % Tween 20 und 2% BSA (PBS-TB).

96-well-ELISA-Platen (Nunc-Immuno Plates) wurden beschichtet mit 100 $\mu$l/well anti-GBP-1 Hybridomaüberstand (im Verhältnis 1:5 mit PBS verdünnt) oder mit gereinigtem Rezeptor in der Konzentration von 1-5 $\mu$g/ml (Inkubation für 16 h bei 4°C). Die Platten wurden mit PBS-T gewaschen und geblockt mit PBS-TB für mindestens 30 min bei Raumtemperatur. Die wells wurden abgesaugt und als Dubletten für 2 h bei Raumtemperatur inkubiert mit 100 $\mu$l des Standards (GBP-1-His, verdünnt in Zellkulturmedium enthaltend 5% FBS), der gleichen Konzentration von BSA als Kontrolle oder mit 100 $\mu$l einer Probe in geeigneter Verdünnung (verdünnt in PBS). Die wells wurden vier mal mit PBS-T gewaschen und mit 100 $\mu$l eines polyklonalen Antikörpers gegen GBP-1, verdünnt in 1:500 in PBS-TB für 2 h bei Raumtemperatur inkubiert. Anschließend wurden die wells wurden vier mal mit PBS-T gewaschen und mit 100 $\mu$l einer

alkalinen Phosphatase, die an einen anti-Kaninchen Antikörper konjugiert ist (Zymed, Berlin, Germany), verdünnt 1:500 in PBS-TB, für 1 h bei Raumtemperatur inkubiert. Anschließend wurden die wells wurden vier mal mit PBS-T gewaschen und mit 100 μl von p-Nitrophenyl Phosphat (Zymed) inkubiert. Die Absorption wurde bei 405 nm in einem "microplate reader" (BioRad) bestimmt. Die Konzentration von GBP-1 in der Probe wurde mit Hilfe der Standardkurve berechnet. Das Verfahren zeigte eine Linearität von 0,1 bis 100 ng/ml von GBP-1/well. Die Variabilität der Ergebnisse in unterschiedlichen Assays lag zwischen 2,3 und 6%.

Ein Beispiel für das Ergebnis eines Nachweises von GBP-1 in Kulturüberstand durch ELISA ist in Figur 4A dargestellt.

Die Sensitivität des ELISAs wurde anhand einer Verdünnungsreihe von GBP-1-His (ein GBP-1-Protein, das aus Bakterien gereinigt wurde und zum Zwecke der Aufreinigung mit 6 Histidinresten am Carboxyterminus versehen ist) in PBS bestimmt.

Die Sensitivität des ELISA wurde festgelegt als die niedrigste Konzentration an GBP-1-His, bei der der zugehörige Messwert sich signifikant, das heißt um mindestens zwei Standardabweichungen, von dem Messwert unterschied, der beim Ansatz ohne GBP-1-His (plus zwei Standardabweichungen) erhalten wurde. Hierbei wurde die Sensitivität des hier beschriebenen ELISA mit 12,3 ng/ml bestimmt.

1. Intra-Assay-Variabilität.

Die Reproduzierbarkeit der Ergebnisse innerhalb eines Versuchs wurde durch dreifach Messungen bestimmt. Dabei wurde die Variabilität auf folgende Weise berechnet:

$$\text{Variabilität} = (\text{Standardabweichung} / \text{Mittelwert}) \times 100\ \%.$$

Es wurden durch Zugabe von GBP-1-His in Serum (1:2 in PBS verdünnt) eines gesunden Probanden drei Testlösungen mit unterschiedlichen GBP-1-His Konzentrationen (400 ng/ml, 180 ng/ml, 40 ng/ml) erzeugt. Mit jeder Lösung wurde die Intra-Assay-Variabilität bestimmt:

GBP-1-His 400 ng/ml; Intra-assay Variabilität = 2,7 %
GBP-1-His 180 ng/ml; Intra-assay Variabilität = 2,8 %
GBP-1-His 40 ng/ml; Intra-assay Variabilität = 2,0 %

2. Inter-Assay-Variabilität:

Zur Bestimmung der Inter-Assay-Variabilität wurde die Reproduzierbarkeit des ELISA in verschiedenen Versuchen bestimmt. Hierzu wurde jede der oben beschriebenen Lösungen sechsmal gemessen. Die inter-Assay-Variabilität wurde wie oben beschrieben berechnet.

GBP-1-His 400 ng/ml; Inter-assay Variabilität = 4,4 %
GBP-1-His 180 ng/ml; Inter-assay Variabilität = 2,8%
GBP-1-His 40 ng/ml; Inter-assay Variabilität = 3,0%

Literature

Carmeliet. and Jain: Nature 407(6801): 249-57 (2000).
Folkman: Nat Med 1(1): 27-31 (1995).
Guenzi, Töpolt, Cornali, Lubeseder-Martellato, Jörg, Matzen, Zietz, Kremmer, Nappi, Schwemmle, Hohenadl, Barillari, Tschachler, Monini, Ensoli, and Stürzl: (2001) EMBO J 20(20): 5568-77..
Guenzi E, Töpolt K, Lubeseder-Martellato C, Jörg A, Naschberger E, Benelli R, Albini A, Stürzl M: (2003) The guanylate binding protein-1 GTPase controls the invasive and angiogenic capability of endothelial cells through inhibition of MMP-1 expression. EMBO J. 22(15):3772-82
Harlow und Lane, "Antibodies, a laboratory manual", CSH Press 1988, Cold Spring Harbor
Töpolt, Guenzi, Lubeseder-Martellato, Jörg, Naschberger, Stürzl: Proceedings of the 22nd Meeting of the European Society of Microcirculation (2002)..
Lubeseder-Martellato, Guenzi, Jörg, Töpolt, Naschberger, Kremmer, Zietz, Tschachler, Hutzler, Schwemmle,

**EP 1 583 970 B1**

Matzen, Grimm, Ensoli and Stürzl: Am J Pathol 161(5): 1749-59 (2002).
Prakash, Praefcke, Renault, Wittinghofer and Herrmann: Nature 403(6769): 567-71 (2000).

**Patentansprüche**

1. In vitro Verfahren zur Identifizierung und/oder Quantifizierung von GuanylatBindungsprotein-1 (GBP-1) oder von Fragmenten dieses Proteins im Kulturüberstand einer Gewebeprobe, einer Probe von Körperflüssigkeit oder einer Probe eines Zellkulturüberstandes, wobei das Verfahren die folgenden Schritte umfasst:

   (a) In Kontakt bringen der Probe mit einem ersten Rezeptor, der GBP-1 spezifsch bindet, wobei der erste Rezeptor ein Antikörper, ein Fab-, Fv- oder scFv-Fragment eines Antikörpers oder ein Aptamer ist, und
   (b) Nachweis einer spezifischen Bindung des Rezeptors mit GBP-1 oder dem Fragment dieses Proteins, wobei das Fragment durch Spaltung entsteht und indikativ für inflammatorische Erkrankungen ist

2. Verfahren nach Anspruch 1, darüber hinaus umfassend den Schritt (a') oder (a") vor dem in Kontakt bringen mit dem ersten Rezeptor

   (a') Markieren der in der Probe enthaltenen Proteine; oder
   (a") Markieren des ersten Rezeptors.

3. Verfahren nach Anspruch 1 oder 2, wobei der Rezeptor vor dem in Kontakt bringen mit GBP-1 oder von Fragmenten dieses Proteins auf einer Oberfläche immobilisiert wird.

4. Verfahren nach Anspruch 1 oder 2, wobei der Rezeptor nach dem in Kontakt bringen mit GBP-1 oder von Fragmenten dieses Proteins auf einer Oberfläche immobilisiert wird.

5. Verfahren nach einem der Ansprüche 3 und 4, wobei das Material der Oberfläche ausgewählt ist aus einer Gruppe bestehend aus Sepharose, Latex, Glas, Polystyrol, Polyvinyl, Nitrocellulose und Silicium.

6. Verfahren nach einem der Ansprüche 3 bis 5, wobei die Oberfläche eine Membran, ein Kügelchen, ein Chip oder eine Platte ist.

7. Verfahren nach Anspruch 6, darüber hinaus umfassend den Schritt (a'") vor dem Schritt des Nachweises einer spezifischen Bindung:

   (a'") Präzipitieren der Kügelchen mit den daran gebundenen Komplexen aus erstem Rezeptor und GBP-1 oder eines Fragmentes dieses Proteins.

8. Verfahren nach Anspruch 7, wobei der Nachweis der spezifischen Bindung in Schritt (b) eine gelelektrophoretische Auftrennung, optional darüber hinaus eine Western-Blot-Analyse umfasst.

9. Verfahren nach einem der Ansprüche 1 bis 8, wobei zum Nachweis einer spezifischen Bindung von GBP-1 oder eines Fragmentes dieses Proteins an den ersten Rezeptor in Schritt (a) die Probe mit einem zweiten Rezeptor für GBP-1 in Kontakt gebracht wird, der an ein Epitop von GBP-1 oder eines Fragmentes dieses Proteins bindet, das nach Bindung des ersten Rezeptors an GBP-1 oder eines Fragmentes dieses Proteins zugänglich ist, und wobei der zweite Rezeptor ein Antikörper, ein Fab-, Fv- oder scFv-Fragment eines Antikörpers oder ein Aptamer ist.

10. Verfahren nach Anspruch 9, wobei der zweite Rezeptor für GBP-1 oder Fragmente dieses Proteins markiert ist.

11. Verfahren nach Anspruch 10, wobei die Markierung des zweiten Rezeptors für GBP-1 oder eines Fragmentes dieses Proteins ein signalgebendes System umfasst oder durch einen weiteren, dritten Rezeptor, der ein signalgebendes System umfasst, spezifisch erkannt wird, wobei der dritte Rezeptor ein Antikörper, ein Fab-, Fv- oder scFv-Fragment eines Antikörpers oder ein Aptamer ist.

12. Verfahren nach Anspruch 11, wobei das signalgebende System ein Enzym umfasst, das dieses Signal abgibt.

13. Verfahren nach einem der Ansprüche 1 bis 12, wobei das Verfahren ein ELISA, EIA, oder RIA ist.

**14.** Verfahren nach einem der Ansprüche 1 bis 13, wobei das Verfahren automatisiert ausgeführt wird.

**Claims**

**1.** In vitro method for identification and/or quantification of guanylate binding protein-1 (GBP-1) or of fragments of this protein in the culture supernatant of a tissue sample, a sample of a body fluid or a sample of a cell culture supernatant, wherein the method comprises the following steps:

(a) contacting the sample with a first receptor which specifically binds GBP-1, wherein the first receptor is an antibody, a Fab, Fv or scFv fragment of an antibody or an aptamer, and
(b) detecting a specific binding of the receptor with GBP-1 or the fragment of this protein, wherein the fragment results from cleavage and is indicative of inflammatory diseases.

**2.** The method according to claim 1, furthermore comprising step (a') or (a") prior to contacting with the first receptor:

(a') labelling the proteins contained in the sample; or
(a") labelling the first receptor.

**3.** The method according to claim 1 or 2, wherein the receptor is immobilised on a surface prior to contacting with GBP-1 or fragments of this protein.

**4.** The method according to claim 1 or 2, wherein the receptor is immobilised on a surface after contacting with GBP-1 or fragments of this protein.

**5.** The method according to any one of claims 3 or 4, wherein the material of the surface is selected from a group consisting of Sepharose, latex, glass, polystyrene, polyvinyl, nitrocellulose and silicon.

**6.** The method according to any one of claims 3 to 5, wherein the surface is a membrane, a bead, a chip or a plate.

**7.** The method according to claim 6, furthermore comprising the step (a"') prior to the step of detecting a specific binding:

(a"') precipitating the beads with the complexes which are bound thereto of the first receptor and GBP-1 or a fragment of this protein.

**8.** The method according to claim 7, wherein the detection of the specific binding in step (b) comprises a gel electrophoretic separation, optionally, furthermore, a Western blot analysis.

**9.** The method according to any one of claims 1 to 8, wherein for the detection of a specific binding of GBP-1 or a fragment of this protein with the first receptor in step (a), the sample is contacted with a second receptor for GBP-1, which binds to an epitope of GBP-1 or a fragment of this protein, which is accessible after the binding of the first receptor to GBP-1 or a fragment of this protein, and wherein the second receptor is an antibody, a Fab, Fv or scFv fragment of an antibody or an aptamer.

**10.** The method according to claim 9, wherein the second receptor for GBP-1 or fragments of this protein is labelled.

**11.** The method according to claim 10, wherein the label of the second receptor for GBP-1 or a fragment of this protein comprises a signalling system or is specifically recognised by a further, third receptor comprising a signalling system, wherein the third receptor is an antibody, a Fab, Fv or scFv fragment of an antibody or an aptamer.

**12.** The method according to claim 11, wherein the signalling system comprises an enzyme emitting the signal.

**13.** The method according to any one of claims 1 to 12, wherein the method is an ELISA, an EIA or an RIA.

**14.** The method according to any one of claims 1 to 13, wherein the method is carried out in an automated way.

**Revendications**

1.  Procédé *in vitro* d'identification et/ou de quantification de la protéine de liaison au guanylate 1 (GBP-1) ou de fragments de cette protéine dans le surnageant de culture d'un échantillon de tissus, d'un échantillon de fluide corporel ou d'un échantillon de surnageant de culture cellulaire, ledit procédé comprenant les étapes suivantes de:

    (a) mise en contact de l'échantillon avec un premier récepteur, qui lie spécifiquement la GBP-1, ledit premier récepteur étant un anticorps, un fragment Fab, Fv ou scFv d'un anticorps ou un aptamère, et
    (b) détection d'une liaison spécifique du récepteur avec GBP-1 ou le fragment de cette protéine, ledit fragment étant produit par clivage et étant indicatif de maladies inflammatoires.

2.  Procédé selon la revendication 1, comprenant en outre avant la mise en contact avec le premier récepteur, l'étape (a') ou (a") de :

    (a') marquage des protéines contenues dans l'échantillon ; ou
    (a") marquage du premier récepteur.

3.  Procédé selon la revendication 1 ou 2, dans lequel le récepteur est immobilisé sur une surface avant la mise en contact avec la GBP-1 ou des fragments de cette protéine.

4.  Procédé selon la revendication 1 ou 2, dans lequel le récepteur est immobilisé sur une surface après la mise en contact avec la GBP-1 ou des fragments de cette protéine.

5.  Procédé selon l'une des revendications 3 et 4, dans lequel la matière de la surface est choisie dans un groupe constitué par le sépharose, le latex, le verre, le polystyrène, le polyvinyle, la nitrocellulose et le silicium.

6.  Procédé selon l'une des revendications 3 à 5, dans lequel la surface est une membrane, une bille, une puce ou une plaque.

7.  Procédé selon la revendication 6, comprenant en outre avant l'étape de détection d'une liaison spécifique, l'étape (a''') de :

    (a''') précipitation des billes avec les complexes qui y sont liés constitués d'un premier récepteur et de la GBP-1 ou d'un fragment de cette protéine.

8.  Procédé selon la revendication 7, dans lequel la détection de la liaison spécifique à l'étape (b) comprend une séparation sur gel d'électrophorèse, éventuellement en outre, une analyse Western-Blot.

9.  Procédé selon l'une des revendications 1 à 8, dans lequel, pour la détection d'une liaison spécifique de la GBP-1 ou d'un fragment de cette protéine au premier récepteur à l'étape (a), l'échantillon est mis en contact avec un deuxième récepteur pour la GBP-1, lequel se lie à un épitope de GBP-1 ou d'un fragment de cette protéine, qui est accessible après liaison du premier récepteur à la GBP-1 ou à un fragment de cette protéine, et dans lequel le deuxième récepteur est un anticorps, un fragment Fab, Fv ou scFv d'un anticorps ou un aptamère.

10. Procédé selon la revendication 9, dans lequel le deuxième récepteur pour la GBP-1 ou des fragments de cette protéine est marqué.

11. Procédé selon la revendication 10, dans lequel le marquage du deuxième récepteur pour la GBP-1 ou un fragment de cette protéine comprend un système émettant un signal ou est reconnu spécifiquement par un autre, troisième récepteur qui comprend un système émettant un signal, dans lequel le troisième récepteur est un anticorps, un fragment Fab, Fv ou scFv d'un anticorps ou un aptamère.

12. Procédé selon la revendication 11, dans lequel le système émettant un signal comprend une enzyme qui émet ce signal.

13. Procédé selon l'une des revendications 1 à 12, dans lequel le procédé est un procédé ELISA, EIA ou RIA.

14. Procédé selon l'une des revendications 1 à 13, dans lequel le procédé est exécuté de manière automatisée.

**Figur 1**

A

B

**Figur 2**

## A

(K-Vektor) [GAG][SV40][PURO]

(AS-Vektor) [GAG][GBP-1][SV40][PURO]

(GBP-1-Vektor) [GAG][GBP-1][SV40][PURO]
1          592

## B

IL-1β

K   AS   GBP-1   K   AS   GBP-1

GBP-1

Aktin

## C

## D

**Figur 3**

16

## Nachweis von sezerniertem GBP-1
## mittels ELISA

**Figur 4**

# Nachweis von zirkulierendem GBP-1 im Plasma IFN-α-behandelter Patienten

**Figur 5**

## Sekretion von GBP-1 aus INF-γ-behandelten HUVEC beruht nicht auf einer Zunahme von Apoptose und/oder Zellpermeabilität

**Figur 6**

A

B

**Figur 7**

**Figur 8**

**Figur 9**

| Median (relative Einheiten) | Kontrollen N=20 13,3 | SLE N=5 46,1 | Arthritis N=5 58,2 | Erysipel N=8 8,6 |
|---|---|---|---|---|

**Figur 10**

| Median (relative Einheiten) | Kontrollen N=9 36,6 | Meningitis N=8 105,8 |
|---|---|---|

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 8903041 A
- WO 8903042 A
- WO 8602736 A
- WO 9731269 A
- US 5928868 A
- US 5242902 A
- US 5468651 A
- US 5547853 A
- US 5616562 A
- US 5641690 A
- US 4956303 A
- US 5928643 A

**In der Beschreibung aufgeführte Nicht-Patentliteratur**

- **LOWMAN.** *Annu. Rev. Biophys. Biomol. Struct.,* 1997, vol. 26, 401-24
- **VON HARLOW ; LANE.** Antibodies, A laboratory manual. Cold Spring Harbor Laboratory, 1988
- **CARMELIET ; JAIN.** *Nature,* 2000, vol. 407 (6801), 249-57
- **FOLKMAN.** *Nat Med,* 1995, vol. 1 (1), 27-31
- **GUENZI ; TÖPOLT ; CORNALI ; LUBESEDER-MARTELLATO ; JÖRG ; MATZEN ; ZIETZ ; KREMMER ; NAPPI ; SCHWEMMLE.** *EMBO J,* 2001, vol. 20 (20), 5568-77
- **GUENZI E ; TÖPOLT K ; LUBESEDER-MARTELLATO C ; JÖRG A ; NASCHBERGER E ; BENELLI R ; ALBINI A ; STÜRZL M.** The guanylate binding protein-1 GTPase controls the invasive and angiogenic capability of endothelial cells through inhibition of MMP-1 expression. *EMBO J.,* 2003, vol. 22 (15), 3772-82
- **HARLOW ; LANE.** Antibodies, a laboratory manual. Cold Spring Harbor, 1988
- **TÖPOLT ; GUENZI ; LUBESEDER-MARTELLATO ; JÖRG ; NASCHBERGER ; STÜRZL.** *Proceedings of the 22nd Meeting of the European Society of Microcirculation,* 2002
- **LUBESEDER-MARTELLATO ; GUENZI ; JÖRG ; TÖPOLT ; NASCHBERGER ; KREMMER ; ZIETZ ; TSCHACHLER ; HUTZLER ; SCHWEMMLE.** *Am J Pathol,* 2002, vol. 161 (5), 1749-59
- **PRAKASH ; PRAEFCKE ; RENAULT ; WITTINGHOFER ; HERRMANN.** *Nature,* 2000, vol. 403 (6769), 567-71